# EUROPÄISCHE PATENTANMELDUNG

(11) **EP 4 785 913 A1**
(43) Veröffentlichungstag der Anmeldung: **05.08.2026**
(21) Anmeldenummer: 25154647.9
(22) Anmeldetag: 29.01.2025
(51) Int. Cl.: A61F 5/02

(54) **EXOSKELETT MIT FLEXIBLEM VERBINDUNGSMITTEL**

(71) Anmelder: Hilti Aktiengesellschaft, 9494 Schaan (LI)
(72) Erfinder: Palloni, Sara, 9470 Buchs (CH); Wörz, Sonya, 86929 Penzing (DE); Schaefer, Martin, 6858 Schwarzach (AT); Duhr, Katharina, 76133 Karlsruhe (DE)
(74) Vertreter: Hilti Aktiengesellschaft Corporate Intellectual Property

(57) **Zusammenfassung**

Die vorliegende Erfindung betrifft ein Exoskelett (200) zur körperlichen Unterstützung eines Anwenders, wobei das Exoskelett (200) folgendes aufweist: einen ersten Schulterträger (230), wobei der erste Schulterträger (230) ein erstes Ende, welches über ein erstes Gurtband (242) mit einem Rückenteil (218) verbunden ist, und ein zweites Ende aufweist; einen zweiten Schulterträger (232), wobei der zweite Schulterträger (232) ein erstes Ende, welches über ein zweites Gurtband (244) mit dem Rückenteil (218) verbunden ist, und ein zweites Ende aufweist, wobei das Exoskelett (200) ein flexibles Verbindungsmittel (250) aufweist, welches die zweiten Enden der ersten und zweiten Schulterträger (230, 232) in Schubrichtung komprimierbar mit dem Rückenteil (218) verbindet.

## Beschreibung

Die vorliegende Erfindung betrifft ein Exoskelett, insbesondere aber nicht ausschließlich ein passives Exoskelett, zur körperlichen Unterstützung eines Anwenders. Das Exoskelett eignet sich insbesondere zur Unterstützung von Arbeitern in der Baubranche.

Exoskelette verbessern den Komfort der Mitarbeiter insbesondere auf der Baustelle und können den durch körperlich anstrengende Arbeit verursachten Krankenstand verringern. Sie sind ideal für kontinuierliche oder sich wiederholende Arbeiten mit und ohne Werkzeug.

Auf dem Markt erhältlich sind Exoskelette, die zur passiven und/oder aktiven Unterstützung der eines Anwenders, insbesondere der Arme, der Beine oder des Rückens dienen. Aktive Exoskelette verfügen über integrierte, motorisierte Aktuatoren, die den Benutzer mit einer erforderlichen Kraft oder einem notwendigen Drehmoment unterstützen. Daher werden sie auch als motorisierte Exoskelette bezeichnet. Typischerweise kommen Elektromotoren als Antriebe zum Einsatz, es ist aber auch bekannt pneumatische oder hydraulische Systeme zum Einsatz zu bringen. Im Gegensatz dazu enthalten passive Exoskelette keine derartigen "aktiven" Aktuatoren. Sie nutzen stattdessen Federn und ihr Gestell, um den Benutzer mit Hilfe von Rückstellkräften zu unterstützen.

Passive Exoskelette verzichten auf aktive Komponenten wie Motoren und elektrische Energieversorgung. Daher sind sie in der Regel kleiner, leichter und weniger hinderlich. Sie sind einfacher zu bedienen, wartungsärmer, benötigen keine Batterieladung und sind kostengünstiger. Insbesondere passive Exoskelette bestehen typisch aus einem textilen Gestell (Soft-Exoskelett), das Komfort und Bewegungsfreiheit bietet. Sie sind auch deshalb eine Verbindung fortschrittlichster Exoskelett-Technologie und Arbeitskleidung.

Soft-Exoskelette auf dem Markt haben durchgängige Schulterträger, welche sich vom Brustbereich bis zum Abschluss am Rücken erstrecken. Beim Vorwärtsbeugen mit geradem Rücken kann es passieren, dass sich die Schulterträger zusammenschieben und derart verformen, dass diese unangenehm in den Rücken des Benutzers drücken. Je länger der Träger, z.B. bei großen Exoskeletten (für große Benutzer), oder je schmäler der Schulterträger, desto stärker tritt dieser Effekt in Erscheinung.

Es ist die Aufgabe der vorliegenden Erfindung, ein verbessertes Exoskelett zur körperlichen Unterstützung eines Anwenders bereitzustellen, welches die oben genannten Nachteile ausräumt und insbesondere ein Zusammenschieben der Schulterträger beim Beugen des Nutzers zu verhindern. Dabei soll die erfindungsgemäße Lösung von der Länge und Breite der Schulterträger unabhängig sein, d.h. auch bei langen und schmalen Trägern ein Zusammenschieben der Träger wirkungsvoll verhindern.

Die oben genannte Aufgabe wird durch den Gegenstand des unabhängigen Patentanspruchs 1 gelöst. Vorteilhafte Weiterbildungen des erfindungsgemäßen Exoskeletts sind in den abhängigen Patentansprüchen angegeben.

Dementsprechend betrifft die vorliegende Erfindung ein Exoskelett zur körperlichen Unterstützung eines Anwenders, wobei das Exoskelett folgendes aufweist:
- einen ersten Schulterträger, wobei der erste Schulterträger ein erstes Ende, welches über ein erstes Gurtband mit einem Rückenteil verbunden ist, und ein zweites Ende aufweist;
- einen zweiten Schulterträger, wobei der zweite Schulterträger ein erstes Ende, welches über ein zweites Gurtband mit dem Rückenteil verbunden ist, und ein zweites Ende aufweist,
wobei das Exoskelett ein flexibles Verbindungsmittel aufweist, welches die zweiten Enden der ersten und zweiten Schulterträger in Schubrichtung komprimierbar mit dem Rückenteil verbindet.

Durch das erfindungsgemäße flexible Verbindungsmittel wird ein Pufferbereich zwischen den Schulterträgern und dem Rückenteil bereitgestellt. Innerhalb dieses Bereichs können die Schulterträger auf den Rückenteil zu geschoben werden, ohne dass Stauchungskräfte in die Schulterträger eingebracht werden. Durch die freie Bewegung im Pufferbereich wird somit sichergestellt, dass ein Aufwerfen der Schulterträger wirkungsvoll verhindert wird. Träger verbleiben somit auch beim Beugen flach am Rücken des Benutzers und drücken nicht gegen diesen. Dies erhöht den Tragekomfort erheblich und verbessert die Standzeit des erfindungsgemäßen Exoskeletts, da Stauchungen an den Schulterträgern verhindert werden.

Nach einer weiteren Ausführungsform ist das flexible Verbindungsmittel in Schubrichtung im Wesentlichen widerstandslos komprimierbar. Durch den fehlenden Widerstand hindert das flexible Verbindungsmittel bzw. das damit ausgestattete Exoskelett den Nutzer in keiner Weise am Beugen. Das Exoskelett kann somit fast unbemerkt getragen werden.

Nach einer weiteren Ausführungsform verbindet das flexible Verbindungsmittel die zweiten Enden der ersten und zweiten Schulterträger in Zugrichtung im Wesentlichen fest mit dem Rückenteil. Gemäß dieser Ausführungsform begrenzt das flexible Verbindungsmittel eine Bewegung der Schulterträger in Zugrichtung. Dies ist vorteilhaft, da die Schulterträger an ihrem dem zweiten Ende gegenüberliegenden, ersten Ende wiederum mit dem Rückenteil verbunden werden, um Schulterschlaufen zum Tragen des Exoskeletts auszubilden. Mit anderen Worten, das flexible Verbindungsmittel ermöglicht einerseits eine freie Beugebewegung und stellt andererseits sicher, dass der Oberteil des Exoskeletts fest mit dem Oberkörper des Nutzers verbunden ist.

Nach einer weiteren Ausführungsform weist das flexible Verbindungsmittel ein flexibles Gewebe auf. Die Benutzung eines flexiblen Gewebes ist eine besonders einfache Ausführungsvariante. Beispielsweise kann das flexible Gewebe ein Textil sein, welches zwischen den Schultergurten und dem Rückenteil angeordnet, beispielsweise angenäht, ist. Die Textilien sollten jedoch fein genug sein, um ein im Wesentlichen widerstandsloses Komprimieren, d. h. Falten, zu erlauben.

Nach einer weiteren Ausführungsform ist das flexible Verbindungsmittel elastisch oder unelastisch ausgebildet. Sollte das flexible Verbindungsmittel elastisch ausgebildet sein (z.B. elastisches Textil), so ist es bevorzugt, dass das elastische Verbindungsmittel in einem ersten Zustand, d.h. wenn der Nutzer aufrecht steht, gespannt vorliegt. Mit anderen Worten ist das flexible Gewebe im ersten Zustand elastisch vorgespannt und versucht die zweiten Enden der Schulterträger auf bzw. über das Rückenteil zu ziehen. Dies ist jedoch erst dann möglich, wenn der Nutzer sich beugt, insbesondere mit geradem Rücken beugt. Beim Beugen verkürzt sich der Abstand zwischen den Zeiten Enden der Schulterträger und dem Rückenteil. Dabei wird das flexible Gewebe des Verbindungsmittels in einen zweiten, komprimierten Zustand überführt. Im Fall des elastischen Verbindungsmittels, zieht sich dieses zusammen und reduziert seine Vorspannung. Durch das elastische Gewebe wirft sich das flexible Verbindungsmittel beim Beugen noch weniger auf. Darüber hinaus ergibt sich durch die elastische Vorspannung eine bessere Führung des Verbindungsmittels zwischen dem ersten Zustand und dem zweiten Zustand. Es wird durch das Zusammenziehen des elastischen Verbindungsmittels bewirkt, dass dieses den Rückenteil im zweiten, komprimierten Zustand besser überlappt.

Nach einer weiteren Ausführungsform ist das flexible Verbindungsmittel an einem ersten Ende mit dem Rückenteil und an einem gegenüberliegenden, zweiten Ende mit mindestens einem Schulterträger verbunden, wobei das erste Ende des flexiblen Verbindungsmittels an einer dem Nutzer abgewandten Oberfläche angebracht ist. Hierdurch kann verhindert werden, dass sich das flexible Verbindungsmittel und die Schulterträger beim Komprimieren unter das Rückteil, d.h. zwischen Rückenteil und Rücken des Nutzers, verschiebt. Vorzugsweise ist das flexible Verbindungsmittel am zweiten Ende der Schulterträger auf einer dem Nutzer zugewandten Oberfläche angebracht, z.B. angenäht. Hierdurch wird ein Verschieben der Schulterträger auf bzw. über den Rückenteil während des Beugens weiter begünstigt.

Nach einer weiteren Ausführungsform weist das flexible Verbindungsmittel einen netzartigen Bereich auf. Durch den netzartigen Bereich ist das flexible Verbindungsmittel besonders atmungsaktiv. Hierdurch wird der Tragekomfort weiter erhöht.

Nach einer weiteren Ausführungsform ist das flexible Verbindungsmittel faltbar. Wie oben bereits erwähnt, kann das flexible Verbindungsmittel ein flexibles Gewebe sein, welches sich bei Komprimierung wulstartig zusammendrückt bzw. faltet. Mit anderen Worten, das flexible Verbindungsmittel kann sich mehrfach übereinander falten, um vom expandierten Grundzustand in den komprimierten Zustand überführt zu werden. In einem weiteren Ausführungsbeispiel kann das flexible Verbindungsmittel Schwächungsbereiche aufweisen, welche als vordefinierte Faltkanten des Verbindungsmittels dienen. Hierdurch wird der Widerstand gegen ein Verschieben der Schulterträger noch einmal reduziert. Auch lässt sich hierdurch das Kompressionsverhalten des flexiblen Verbindungsmittels besser steuern, sodass dieses nahe am Exoskelett verbleibt und somit vor Beschädigungen geschützt ist.

Nach einer weiteren Ausführungsform ist das flexible Verbindungsmittel von einem ersten, gespannten Zustand, insbesondere bei Aufrechter Haltung des Nutzers, in einen zweiten, komprimierten Zustand, insbesondere bei gebeugter Haltung des Nutzers, überführbar.

Nach einer weiteren Ausführungsform weist das Exoskelett verstellbare Schultergurte zum Anpassen des Exoskeletts an die Größe des Benutzers auf, wobei die verstellbaren Schultergurte insbesondere durch die Schulterträger geführt werden. Da die Schultergurte gemäß dieser Ausführungsform durch die Schulterträger geführt werden, hat das flexible Verbindungsmittel gemäß der vorliegenden Erfindung weiterhin den Vorteil, dass die Spannung auf den verstellbaren Schultergurten nicht durch ein Verbiegen der Schulterträger beim Beugen beeinflusst wird. Bei aus dem Stand der Technik bekannten Exoskeletten ohne flexible Verbindungsmittel kann es dazu kommen, dass durch die oben bereits erwähnten Aufwerfungen der Schulterträger zusätzliche Spannung auf den Schultergurten lastet. Diese Spannungen können zu zusätzlichen Rückstellkräften im Expander führen, welche seitens des Herstellers nicht kalkulierbar sind.

Nach einer weiteren Ausführungsform weist das Exoskelett wenigstens einen elastischen Expander auf, welcher zwischen wenigstens einem der verstellbaren Schultergurte des Exoskeletts und wenigstens einem Beingurt angeordnet und dazu ausgebildet ist, eine Rückstellkraft zu erzeugen, wenn der Anwender, insbesondere durch eine Beugung nach vorne, einen Abstand zwischen dem Schultergurt und dem Beingurt vergrößert. Bei dem Expander handelt es sich um eine elastische Vorrichtung, welche dazu dient, die Beugung des Nutzers in eine potentielle Energie, das heißt eine Streckung des Expanders, umzuwandeln. Die Rückstellkraft des Expanders unterstützt sodann die Gegenbewegung des Nutzers, beispielsweise das Aufrichten.

Nach einer weiteren Ausführungsform weist das Exoskelett zwei Beingurte auf, welche dazu ausgebildet sind, je ein Bein des Benutzers zu umschlingen, und wobei das Exoskelett einen ersten elastischen Expander aufweist, welcher zwischen einem ersten verstellbaren Schultergurt und dem ersten Beingurt angeordnet ist, und wobei das Exoskelett einen zweiten elastischen Expander aufweist, welcher zwischen einem zweiten verstellbaren Schultergurt und dem zweiten Beingurt angeordnet ist.

Nach einer weiteren Ausführungsform ist der erste Beingurt über einen ersten verstellbaren Riemen mit dem ersten Expander verbunden, wobei der zweite Beingurt über einen zweiten verstellbaren Riemen mit dem zweiten Expander verbunden ist.

Nach einer weiteren Ausführungsform ist das Exoskelett dazu ausgebildet, ein Aufrichten des Anwenders passiv zu unterstützen. Wie oben bereits erwähnt, unterscheidet sich das passive Exoskelett vom aktiven Exoskelett insbesondere darin, dass keine aktiven Aktuatoren beziehungsweise Motoren verwendet werden. Vielmehr wird eine Bewegung des Nutzers in potenzielle Energie einer elastischen Vorrichtung gespeichert, um diese bei einer Gegenbewegung des Nutzers wieder freizugeben. Die hierdurch freigegebene, potenzielle Energie der elastischen Vorrichtung (beispielsweise Expander) unterstützt den Nutzer bei der Gegenbewegung und entlastet dessen Muskeln.

Ein weiterer Aspekt der vorliegenden Offenbarung betrifft ein Exoskelett zur körperlichen Unterstützung eines Anwenders, wobei das Exoskelett folgendes aufweist:
- Einen Oberteil, welcher ein Rückenteil und Schulterträger aufweist, wobei der Rückenteil derart mit den Schulterträgern verbunden ist, dass der Oberteil an einem Oberkörper des Nutzers befestigbar ist;
- Einen Unterteil, welcher wenigstens einen Beingurt zum Befestigen des Unterteils an einem Oberschenkel des Nutzers aufweist,
wobei der der Rückenteil über einen Beingurt-Verbinder mit dem wenigstens einen Beingurt verbunden ist, und wobei der wenigstens eine Beingurt eine Werkzeugschlaufe zur Aufnahme von Werkzeug aufweist.

Nach einem weiteren Aspekt weist der Beingurt-Verbinder ein Riemen auf, welcher in der Länger veränderlich ist. Durch den Riemen ist der Beingurt-Verbinder individuell auf die Größe des Nutzers einstellbar, sodass der wenigstens eine Beingurt auf einer angenehmen Position am Oberschenkel getragen werden kann. Durch den Bein-gurt Verbinder wird insbesondere ein Verrutschen des Beingurts verhindert.

Nach einem weiteren Aspekt ist der wenigstens eine Beingurt über wenigstens einen elastischen Expander mit deinem der Schulterträger verbunden.

Weitere Vorteile ergeben sich aus der folgenden Figurenbeschreibung. Die Figuren, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Rückansicht eines Exoskeletts gemäß einer Ausführungsform der vorliegenden Erfindung;
- Fig. 2: eine Frontansicht eines Exoskeletts gemäß einer Ausführungsform der vorliegenden Erfindung, in einer Aufrechten Trageposition;
- Fig. 3: eine Rückansicht des Exoskeletts gemäß Fig. 2, in der Aufrechten Trageposition;
- Fig. 4: eine perspektivische Seitenansicht des Exoskeletts gemäß Fig. 2, in einer gebeugten Trageposition.

### Detaillierte Beschreibung

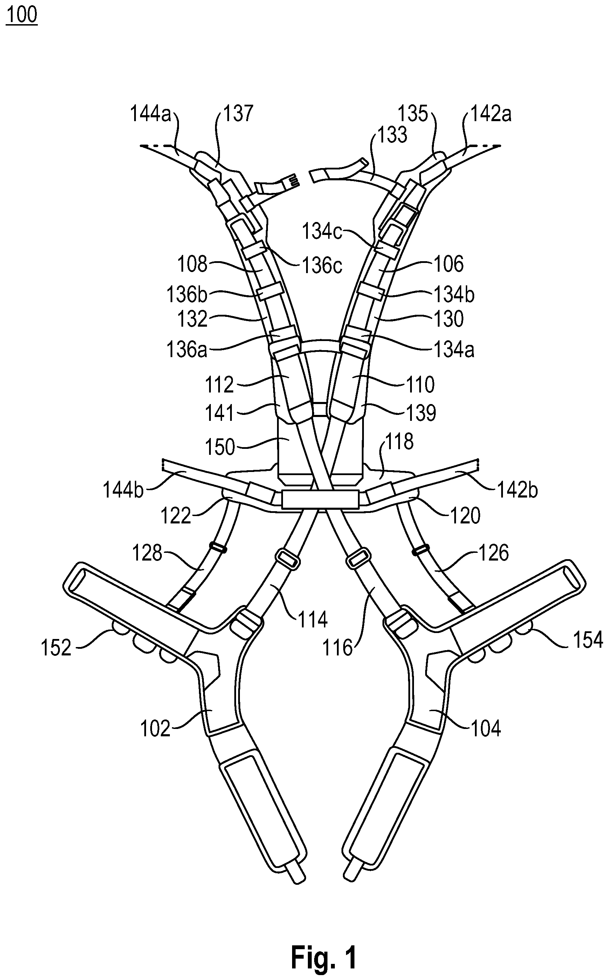

Figur 1 zeigt eine schematische Rückansicht eines Exoskeletts 100 gemäß der vorliegenden Erfindung. Bei dem Exoskelett 100 gemäß Figur 1 handelt es sich um ein passives Exoskelett. Demnach wandelt das Exoskelett Bewegungen des Nutzers in potentielle Energie einer oder mehrerer elastischer Vorrichtungen, um diese bei der Gegenbewegung wieder abzugeben und somit den Nutzer zu unterstützen.

Das Exoskelett 100 weist einen ersten Beingurt, 102 und einen zweiten Beingurt 104 auf. Der erste Beingurt 102 wird zur Befestigung des Exoskeletts 100 an einem ersten Bein des Benutzers angebracht. Der zweite Beingurt 104 wird zur Befestigung des Exoskeletts 100 an einem zweiten Bein des Benutzers angebracht. In der hier dargestellten Rückansicht ist der erste Beingurt 102 für das linke Bein des Benutzers geeignet, während der zweite Beingurt 104 am rechten Bein angebracht wird. Der erste Beingurt 102 und der zweite Beingurt 104 können über einen Klettverschluss um die Beine des Nutzers geschlungen werden, um die Beingurte 102, 104 an den Umfang des jeweiligen Oberschenkels des Benutzers anzupassen. Selbstverständlich können die Beingurte 102, 104 auch anderweitig verstellbar sein, bspw. können diese als Schlaufen mit verstellbaren Riemen ausgebildet sein.

Die Beingurte 102, 104 können darüber hinaus elastisch ausgebildet sein bzw. ein elastisches Element aufweisen, um die Änderung des Oberschenkeldurchmessers bzw. -umfangs während der Bewegungen, z.B. beim Knieen, auszugleichen.

Die Beingurte 102, 104 bilden einen Unterteil des Exoskeletts 100 aus. Ein Oberteil des Exoskeletts 100 erstreckt sich über den oberen Teil des Rückens, die Schultern und den Brustbereich des Nutzers.

Das Oberteil weist insbesondere einen Rückenteil 118 auf, welcher entweder am unteren Ende des Brustkorbs oder im Wesentlichen unterhalb des Brustkorbs zum Liegen kommt, wenn das Exoskelett durch den Nutzer getragen wird. Der Rückenteil 118 ist dabei jedoch immer vom Hüftknochen des Nutzers nach oben beabstandet, d.h. es handelt sich bei dem Rückenteil insbesondere nicht um einen Hüftgurt, wie dies später näher erläutert werden wird. Vom Rückenteil 118 erstrecken sich zwei Schulterträger 130, 132. An einem ersten Ende 135 ist ein erster Schulterträger 130 mit einem ersten Seitenbereich 120 des Rückenteils 118 verbunden. In der hier dargestellten Ausführungsform handelt es sich bei dem ersten Seitenbereich 120 um die rechte Seite des Rückenteils 118. Der zweite Schulterträger 132 ist an seinem ersten Ende 137 mit einem zweiten Seitenbereich 122 des Rückenteils 118 verbunden. In der hier dargestellten Ausführungsform ist der zweite Seitenbereich 122 der linke Bereich des Rückenteils 118. Insbesondere sind die Schulterträger 130, 132 jeweils über verstellbare Riemen 142, 144 mit ihren jeweiligen Seitenbereichen des Rückenteils 118 verbunden.

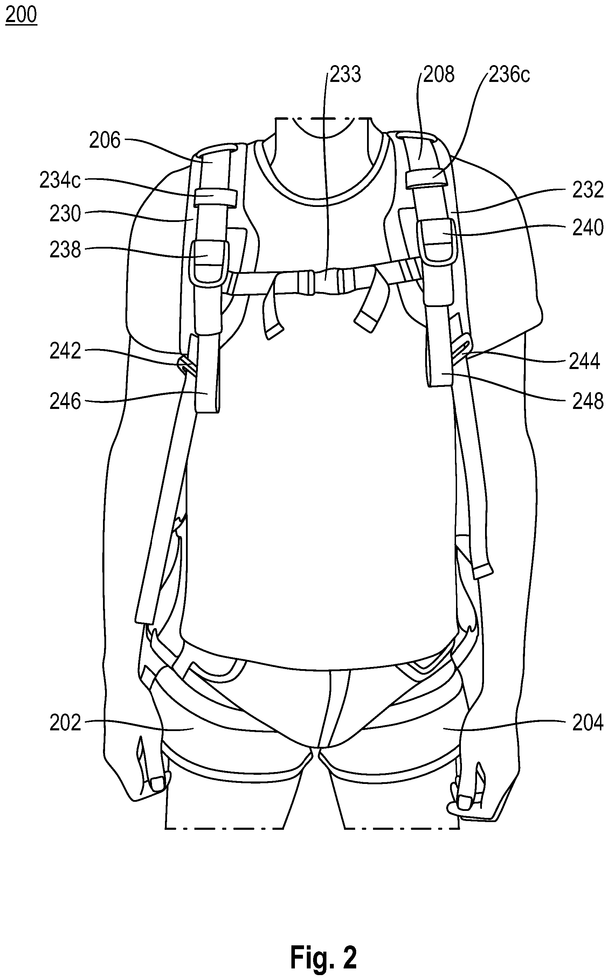

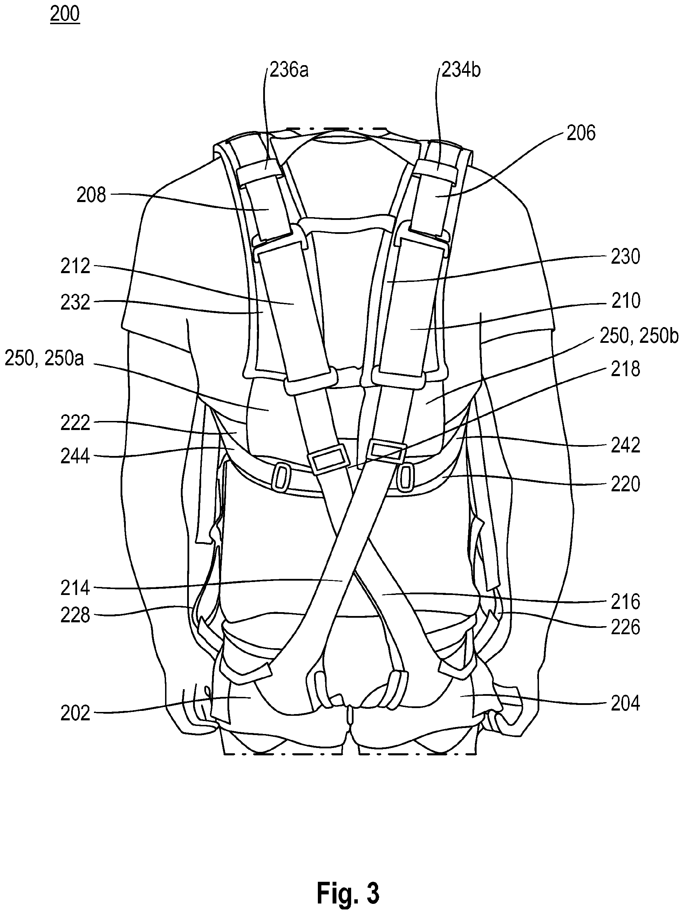

Die Verbindung zwischen den Schulterträgern 130, 132 und dem Rückenteil 118 entspricht im Wesentlichen dem Aufbau eines Rucksacks. In der Figur 1 sind die verstellbaren Riemen 142, 144 zweigeteilt dargestellt. Dies dient der Übersichtlichkeit. Es sei jedoch angemerkt, dass die beiden Endbereiche 142a, 142b des ersten Riemens 142 in Wirklichkeit miteinander verbunden sind. Entsprechend sind auch die beiden Enden 144a, 144b des zweiten Riemens 144 in Wirklichkeit miteinander verbunden und nicht, wie schematisch dargestellt, voneinander getrennt. Dies ist auch aus dem Anwendungsbeispiel gemäß der Figuren 2 und 3 ersichtlich.

Die beiden Schulterträger 130, 132 sind im Brustbereich über einen Schultergurt 133 verbindbar (Fig. 2).

Der erste Schulterträger 132 ist mit einem zweiten Ende 139 an einem oberen Bereich des Rückenteils 118 befestigt. Der zweite Schulterträger 132 ist mit seinem zweiten Ende 141 ebenfalls an einem oberen Bereich des Rückenteils 118 befestigt. Wie dies später näher erläutert werden wird, sind die beiden zweiten Enden 139, 141 über ein flexibles Verbindungsmittel 150 mit dem oberen Bereich des Rückenteils 118 verbunden.

Das flexible Verbindungsmittel 150 ist an einer dem Nutzer abgewandten Oberfläche des Rückenteils 118 befestigt, bspw. angenäht. Hierdurch kann erreicht werden, dass sich die Schulterträger 130, 132 auf bzw. über das Rückenteil 118 verschiebt und nicht darunter. Ein darunter Schieben, das heißt ein Zurückziehen des flexiblen Verbindungsmittels in den Zwischenraum zwischen Rückenteil und Nutzer könnte der Träger spüren. An den zweiten Enden 139, 141 der Schulterträger 130, 132 ist das flexible Verbindungsmittel auf einer dem Nutzer zugewandten Oberfläche befestigt. In der hier dargestellten Ansicht bedeutet dies, dass das Verbindungsmittel 150 auf einer Oberseite des Rückenteils 118 und auf einer Unterseite der Schulterträger 130, 132 angebracht ist.

Das Oberteil ist über zumindest teilweise dehnbare Gurte bzw. Riemen mit dem Unterteil verbunden. Aus dem Stand der Technik ist bekannt, dass durch diese elastisch verformbaren Gurte bzw. Riemen eine Rückstellkraft in das System eingebracht werden kann, wenn der Abstand zwischen dem Oberteil und dem Unterteil des Exoskeletts 100 beim Beugen vergrößert wird. Diese Rückstellkraft unterstützt den Benutzer beim Aufrichten, indem sich die elastischen Gurte bzw. Riemen wieder zusammenziehen und die potentielle Energie zur Unterstützung des Benutzers freigeben.

Bei der in Figur 1 dargestellten Ausführungsform weist die elastische Verbindung zwischen dem Ober- und Unterteil einen ersten und einen zweiten Schultergurt 106, 108 auf. Der erste Schultergurt 106 wird vom ersten Schulterträger 130 geführt. Insbesondere ist der erste Schultergurt 106 auf einer der Haut des Nutzers abgewandten Oberfläche des ersten Schulterträgers 130 angeordnet. In der hier dargestellten Ausführungsform erstreckt sich der erste Schultergurt 106 durch eine Vielzahl von Schlaufen 134a, 134b, 134c, sodass der erste Schultergurt 106 nicht vom ersten Schulterträger 130 rutschen kann. Dennoch kann der erste Schultergurt 106 in Längsrichtung relativ zum Schulterträger 130 verstellt werden, um den Schultergurt 106 auf die Größe des Benutzers anzupassen.

Der zweite Schultergurt 108 wird vom zweiten Schulterträger 132 geführt. Insbesondere ist der zweite Schultergurt 108 auf einer der Haut des Nutzers abgewandten Oberfläche des zweiten Schulterträgers 132 angeordnet. Der zweite Schultergurt 106 erstreckt sich durch eine Vielzahl von Schlaufen 136a, 136b, 136c, sodass der zweite Schultergurt 108 nicht vom zweiten Schulterträger 132 rutschen kann. Dennoch kann der zweite Schultergurt 108 in Längsrichtung relativ zum Schulterträger 132 verstellt werden, um den Schultergurt 108 auf die Größe des Benutzers anzupassen.

Die beiden Schultergurte 106, 108 sind im Wesentlichen nicht elastisch, d. h. sie sind in ihrer Länge nicht elastisch verformbar. Zwischen dem ersten Schultergurt 106 und dem ersten Beingurt 102 ist ein erster Expander 110 vorgesehen. Zwischen dem zweiten Schultergurt 108 und dem zweiten Beingurt 104 ist ein zweiter Expander 112 vorgesehen. Bei den Expandern 110, 112 handelt es sich um elastische Vorrichtungen, welche dazu dienen, die Bewegungen des Nutzers in potentielle Energie (Rückstellkräfte) der Expander umzuwandeln und diese bei einer Gegenbewegung wieder freizugeben. In der hier dargestellten Ausführungsform werden die ersten und zweiten Expander 110, 112 insbesondere dann gestreckt, wenn der Benutzer sich nach vorne beugt. Es sei an dieser Stelle angemerkt, dass statt der Expander jede andere elastische Vorrichtung verwendet werden kann, welche bei Streckung (oder auch Stauchung) eine Rückstellkraft verursacht.

Zwischen dem unteren Ende der Expander, 110, 112 und den Beingurten 102, 104 sind verstellbare Riemen 114, 116 angebracht. Ein erster verstellbarer Riemen 114 ist zwischen dem ersten Beingurt 102 und dem ersten Expander 110 angeordnet. Ein zweiter verstellbarer Riemen 116 ist zwischen dem zweiten Beingurt 104 und dem zweiten Expander 112 angeordnet. Die verstellbaren Riemen 114, 116 sind dazu ausgelegt, das Exoskelett 100 auf die Größe des Nutzers anzupassen. Insbesondere sollten die Riemen 114, 116 derart eingestellt werden, dass diese, bei aufrechter Haltung des Nutzers, straff zwischen den Beingurten 102, 104 und den unteren Enden der Expander 110, 112 verlaufen. Auch die Riemen 114, 116 sind im Wesentlichen nicht elastisch verformbar.

Zwischen dem Rückenteil 118 und den Beingurten 102, 104 sind Beingurt-Verbinder, 128, 126 vorgesehen. Ein erster Beingurt-Verbinder 126 ist zwischen dem Rückenteil 118 und dem ersten Beingurt 102 vorgesehen. Ein zweiter Beingurt-Verbinder 128 ist zwischen dem Rückenteil 118 und dem zweiten Beingurt 104 vorgesehen. Die Beingurt-Verbinder 126, 128 dienen unter anderem dazu, den Rückenteil 118 korrekt am Rücken des Nutzers zu positionieren. Auch verhindern die Beingurt-Verbinder 126, 128 korrekt angelegt ein Verkippen der Beingurte 102, 104 am Oberschenkel des Nutzers.

Das Unterteil Exoskeletts ist im Wesentlich frei beweglich gegenüber dem Oberteil des Exoskeletts. Es ist jedoch von Vorteil, dass sich die Schultergurte am Rücken des Nutzers (hier auf Höhe des Rückenteils 118) kreuzen, um eine optimale Kraftverteilung zu gewährleisten. Hierzu ist es jedoch notwendig, dass die Beingurte 102, 104 jeweils am dafür vorgesehenen Bein befestigt werden. Insbesondere sollte der erste Beingurt 102 mit dem linken Bein des Nutzers und zweite Beingurt 104 mit dem rechten Bein des Nutzers verbunden werden. Die Beingurt-Verbinder stellen sicher, dass der erste Beingurt 102 auch beim Anziehen korrekt gegenüber dem linken Seitenbereich des Rückenteils 118 ausgerichtet ist. Entsprechend bleibt der zweite Beingurt 104 gegenüber dem rechten Seitenbereich des Rückenteils 118 ausgerichtet. Das korrekte Anlegen des Exoskeletts 100 wird also durch die Beingurt-Verbinder 120, 122 vereinfacht.

Aus Figur 1 ist ferner ersichtlich, dass an den Beingurten 102, 104 Werkzeugträger, insbesondere Werkzeugschlaufen 152, 154 angeordnet sind. Am ersten Beingurt 102 sind erste Werkzeugschlaufen 152 angeordnet. Am zweiten Beingurt 104 sind zweite Werkzeugschlaufen 154 angeordnet. Die Werkzeugschlaufen 152, 154 können dazu genutzt werden, Werkzeug oder jeden anderen geeigneten Gegenstand an den Beingurten einzuhängen. Dies ermöglicht es dem Nutzer, seinen Rücken über das Exoskelett 100 zu entlasten und gleichzeitig Werkzeuge und andere Hilfsmittel jederzeit griffbereit zu haben. Dies ist insbesondere deshalb von Vorteil, da Teile des Exoskeletts (z.B. die Beingurte 102, 104) Aufbewahrungsvorrichtungen (z.B. Taschen oder Schlaufen) der darunter liegenden Arbeitskleidung überdecken können, sodass diese nicht mehr zur Aufbewahrung von Werkzeug oder anderen Gegenständen zur Verfügung stehen.

Bei Verwendung der Werkzeugträger kommt den oben genannten Beingurt-Verbinder 126, 128 eine weitere Funktion zu. So wird durch die Beingurt-Verbinder ein ausreichender Halt der Beingurte 102, 104 unter dem Gewicht der Werkzeuge gewährleistet. Die Beingurt-Verbinder verhindern also ein Abrutschen bzw. ein Verdrehen/Verkippen der Beingurte 102, 104.

Bei dem flexiblen Verbindungsmittel 150 gemäß Figur 1 handelt es sich um ein flexibles Gewebe. Insbesondere weist das flexible Verbindungsmittel einen netzartigen Bereich auf. Dieser ist einerseits atmungsaktiv und kann sich andererseits in Schubrichtung, d. h. wenn sich das zweite Ende 139, 141 der Schulterträger 130, 132 auf das Rückenteil 118 zubewegt (beispielsweise beim Beugen), im Wesentlichen ohne Widerstand verformen. Beispielsweise kann das netzartige Gewebe des flexiblen Verbindungsmittels 150 wulstartig über sich selbst gefaltet werden. Mit anderen Worten, das flexible Verbindungsmittel 150 gemäß Figur 1 ermöglicht ein im Wesentlichen freies Komprimieren in Schubrichtung. Dagegen kann das flexible Verbindungsmittel 150 in Zugrichtung, d. h. wenn die zweiten Enden 139, 141 der Schulterträger 130, 132 vom Rückenteil 118 entfernt werden, stabil d.h. unelastisch sein. Insbesondere in Figur 1 ist das Exoskelett 100 in einem Zustand dargestellt, in welchem das flexible Verbindungsmittel 150 voll expandiert ist, d. h. die zweiten Enden 139, 141 der Schulterträger 130, 132 sind maximal vom Rückenteil 118 beabstandet. Dieser erste Zustand ist auch der Ausgangszustand in der aufrechten Haltung des Nutzers, wie dies im Zusammenhang mit Figur 2 und 3 näher erläutert werden wird.

In einer alternativen Ausführungsform kann das flexible Verbindungselement elastisch sein, bspw. aus einem elastischen Gewebe gefertigt werden. In diesem Fall ist es bevorzugt, dass das elastische Verbindungsmittel in der aufrechten Haltung des Nutzers gedehnt vorliegt und sich beim Beugen zusammenzieht. Hierdurch ergibt sich beim Beugen weniger Material, welches bei der Bewegung der Schulterträger 130, 132 auf den Rückenteil 118 zu komprimiert (bzw. gefaltet) werden muss. Die Elastizität des Verbindungsmittels kann also ein unangenehmes Aufwerfen des Verbindungsmittels verhindern.

Das flexible Verbindungsmittel 150 bildet einen Pufferbereich zwischen dem Rückenteil 118 und den Schulterträgern 130, 132 aus. Dieser Pufferbereich gewährleistet, dass ein Verschieben der Schulterträger 130, 132 auf den Rückenteil 118 zu (während dem Beugen) widerstandsfrei möglich ist. Dabei werden keine Schubkräfte in die Schulterträger 130, 132 eingebracht, sodass diese ihre flache Ausrichtung beibehalten (d. h. sie werfen sich nicht auf) und somit nicht in den Rücken des Nutzers drücken. Es sei an dieser Stelle angemerkt, dass bei Exoskeletten nach dem Stand der Technik ein Aufwerfen der Schulterträger insbesondere dann auftreten, wenn sich der Nutzer mit geradem Rücken beugt, bspw. um einen Gegenstand zu heben. Bekannte Exoskelette können also beim korrekten (gesunden) Heben mit geradem Rücken Schmerzen bereiten, die den Nutzer tendenziell von einer korrekten Beugehaltung abhalten. Das neuartige, flexible Verbindungsmittel hat mithin den Vorteil, dass eine korrekte Körperhaltung begünstigt wird.

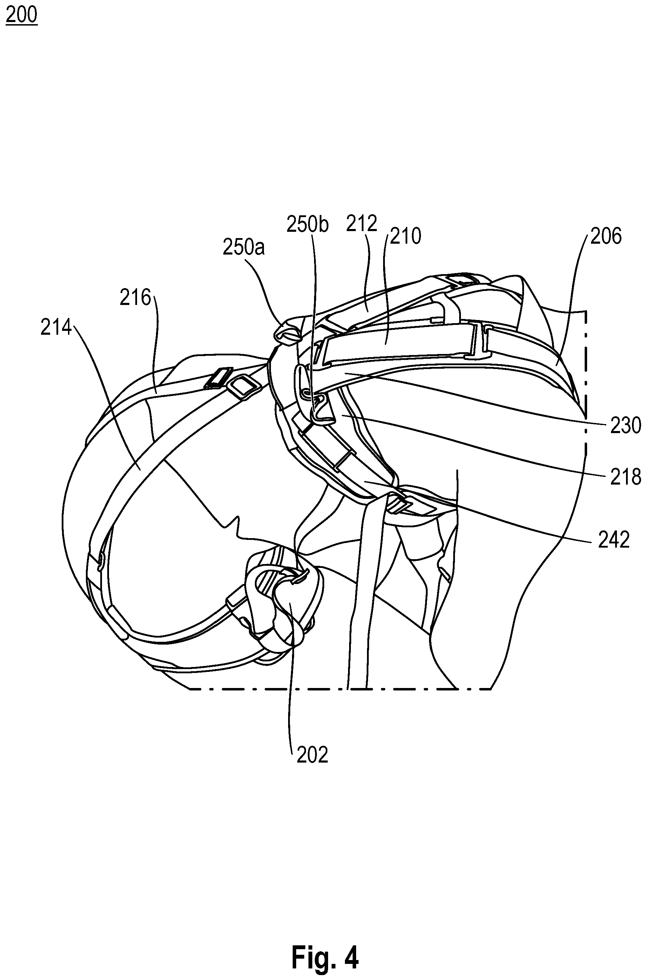

Eine zweite Ausführungsform der vorliegenden Erfindung ist in den Figuren 2 bis 4 dargestellt. In den Figuren 2 und 3 ist das Exoskelett 200 gemäß der zweiten Ausführungsform in einem ersten Zustand dargestellt, in welchem der Nutzer aufrecht steht. In Figur 4 ist ein zweiter Zustand des Exoskeletts dargestellt, in welchem der Benutzer nach vorne gebeugt ist.

Der Aufbau des Exoskeletts 200 gemäß der zweiten Ausführungsform entspricht im Wesentlichen dem Aufbau des Exoskeletts 100 gemäß der ersten Ausführungsform. Um Wiederholungen zu vermeiden, wurden gleichartige bzw. gleich wirkende Bauteile mit entsprechenden Bezugszeichen versehen, welche um "100" erhöht wurden.

Im Unterschied zur Ausführungsform gemäß Figur 1, ist das flexible Verbindungsmittel 250 gemäß der zweiten Ausführungsform nicht als netzartige Struktur ausgebildet. Vielmehr handelt es sich bei dem flexiblen Verbindungsmittel 250 um ein Stoffmaterial, welches die zweiten Enden der Schulterträger 230, 232 mit dem Rückenteil 218 verbindet. Es kann sich bei dem flexiblen Verbindungsmittel 250, also auch um jegliches Textil handeln, welches einerseits in Schubrichtung komprimierbar und andererseits in Zugrichtung formstabil ist.

Auch ist aus Figur 3 ersichtlich, dass das flexible Verbindungsmittel 250 zweiteilig ausgebildet ist. Ein erstes Textil 250a verbindet das zweite Ende des ersten Schulterträger 230 mit dem Rückenteil 218. Ein zweites Textil 250b verbindet das zweite Ende des zweiten Schulterträgers 232 mit dem Rückenteil 218. Die beiden Textilien des flexiblen Verbindungmittels 250 sind dabei voneinander beabstandet. Selbstverständlich kann auch ein einzelnes Textil zwischen den beiden Enden der Schulterträger 230, 232 und dem Rückenteil 218 angeordnet werden. Es ist denkbar, jede Anzahl an Textilien bzw. flexiblen Materialien zu verwenden, um die zweiten Enden der Schulterträger 230, 232 mit dem Rückenteil 218 zu verbinden. So können beispielsweise auch Schnüre oder Federn zwischen den Schulterträgern 230, 232 und dem Rückenteil 218 angeordnet werden.

In Figur 2 ist eine Vorderansicht des Exoskeletts 200 gemäß der zweiten Ausführungsform gezeigt. Wie oben bereits erwähnt, erstrecken sich die Schulterträger 230, 232 von Ihrem zweiten Ende über die Schultern des Benutzers zum ersten Ende, ähnlich eines Rucksacks. Das erste Ende der Schulterträger 230, 232 ist im Brustbereich angeordnet. Die ersten Enden sind über erste und zweite verstellbare Riemen 242, 244 mit gegenüberliegenden, seitlichen Bereichen des Rückenteils 218 (Figur 3) verbunden. Somit ist auch der Rückenteil 218 (Figur 3) etwa auf Brusthöhe, d. h. auf mittlerer Höhe des Rückens, angeordnet. Der Rückenteil 218 ist insbesondere nicht mit einem Hüftgurt zu verwechseln. Die Exoskelette der Figuren 1 bis 4 dargestellten Ausführungsformen weisen keine derartigen Hüftgurte auf. Es wurde festgestellt, dass Exoskelette ohne Hüftgurte einen besseren Tragekomfort bieten. Alternativ kann ein Hüftgurt vorgesehen werden, sollte dies aus anderen Gründen notwendig erscheinen.

Die ersten und zweiten Schultergurte 206, 208 sind auf den Schulterträgern 230, 232 geführt. Die Schultergurte 206, 208 sind in ihrer Länge verstellbar. Hierzu weist das Exoskelett 200 erste und zweite Schnallen, 238, 240 auf. Die Schnallen, 238, 240 sind in Figur 2 in ihrem geschlossenen Zustand dargestellt. Im geschlossenen Zustand der Schnallen 238, 240 kann die Länge der Schultergurte 206, 208 nicht verstellt werden. Durch Öffnen der Schnallen, 238, 240 können die Schultergurte entweder verlängert oder verkürzt werden. Durch Anziehen der Handschlaufen 246, 248 werden die Schultergurte 206, 208 verkürzt. Die Schultergurte werden beim Anziehen des Exoskeletts 200 nach Öffnen der Schnallen derart eingestellt, dass die Expander 210, 212 in der aufrechten Haltung des Nutzers (Figuren 2 und 3) gerade noch nicht bis leicht gespannt sind. Es handelt sich bei dieser Grundspannung der Expander um eine Präferenz des Nutzers, je nachdem wie schnell und stark die gewünschte Unterstützungskraft des Exoskeletts erreicht werden soll. Danach werden die Schnallen geschlossen und müssen nicht mehr betätig werden. Dies bedeutet, dass die Expander 210, 212 elastisch expandiert werden, sobald sich der Nutzer nach vorne beugt (zum Beispiel Figur 4). Es ist dabei grundlegend egal, ob sich der Nutzer mit geradem oder rundem Rücken beugt. Das Exoskelett 200 unterstützt den Nutzer in beiden Positionen. Es wird eine Rückstellkraft erzeugt, welche es dem Nutzer erleichtert, wieder in die aufrechte Position zu gelangen oder auch eine gebeugte Position leichter zu halten. Dies entlastet vor allem den unteren Rücken des Nutzers, z.B. bei schweren bzw. wiederholten Hebebewegungen.

Die Figur 4 zeigt die zweite Ausführungsform des Exoskeletts 200 gemäß der vorliegenden Erfindung in einer gebeugten Stellung des Nutzers. Wie oben bereits erwähnt sind die Expander 210, 212 in dieser Stellung unter Zuglast, sodass diese eine Rückstellkraft bewirken. Durch das Beugen des Nutzers, verkürzt sich die Distanz zwischen dem zweiten Ende der Schulterträger 230, 232 und dem Rückenteil 218. Tatsächlich verschieben sich die Schulterträger 230, 232 im Beispiel gemäß Figur 4 derart, dass die zweiten Enden der Schulterträger 230, 232, in der gebeugten Haltung, den Rückenteil 218 zumindest teilweise überlappen. Ein derartiges Verschieben der Schulterträger 230, 232 gegenüber dem Rückenteil 218 wird insbesondere durch den erfindungsgemäßen, flexiblen Verbindungsbereich 250 ermöglicht. Dieser faltet sich bei der Schubbewegung der Schulterträger 230, 232 zusammen, sodass der Schubbewegung der Schulterträger 32, 232 im Wesentlichen kein Widerstand entgegengesetzt wird. Folglich kommt es auch nicht zum Aufwerfen der Schulterträger 230, 232, sodass diese flach bleiben und den Nutzer nicht beeinträchtigen.

Die vorliegende Erfindung ist nicht auf die in den Figuren dargestellten Ausführungsformen beschränkt, sondern ergibt sich aus einer Zusammenschau sämtlicher hierin offenbarter Merkmale. Insbesondere ist die Erfindung nicht auf die in den Figuren gezeigten Ausführungen des flexiblen Verbindungsmittels beschränkt. Vielmehr ist es ein Kerngedanke der vorliegenden Erfindung, dass das Verbindungsmittel 250 zwar eine freie Bewegung der Schulterträger 230, 232 auf das Rückenteil 218 zu zulässt. Ein Bewegen der Schulterträger 230, 232 gegenüber dem Rückenteil 218 wird jedoch vorzugsweise durch das flexible Verbindungsmittel 250 limitiert. Zu diesem Zweck eignen sich insbesondere Textilien oder Seile bzw. Federn, welche in Schubrichtung im Wesentlichen ohne Widerstand komprimierbar sind. In Zugrichtung sollten diese jedoch im Wesentlichen formstabil sein.

### Bezugszeichenliste

- 100, 200: Exoskelett
- 102, 104, 202, 204: Beingurt
- 106, 108, 206, 208: Schultergurt
- 110, 112, 210, 212: Expander
- 114, 116, 214, 216: verstellbare Riemen
- 118, 218: Rückenteil
- 120, 122, 220, 222: Seitenbereich
- 126, 128, 226, 228: Beingurt-Verbinder
- 130, 132, 230, 232: Schulterträger
- 133: Brustgurt
- 134a, 134b, 134c: Lasche
- 135, 137: erstes Ende
- 139, 141: zweites Ende
- 136a, 136b, 136c: Lasche
- 142a, 142b: Riemen
- 144a, 144b: Riemen
- 150, 250: flexibles Verbindungsmittel
- 152, 154: Werkzeugschlaufen
- 238, 240: Schnalle
- 242, 244: Riemen
- 246, 248: Zuglasche

## Patentansprüche

1. Exoskelett zur körperlichen Unterstützung eines Anwenders, wobei das Exoskelett (200) folgendes aufweist:
- einen ersten Schulterträger (230), wobei der erste Schulterträger (230) ein erstes Ende, welches über ein erstes Gurtband (242) mit einem Rückenteil (218) verbunden ist, und ein zweites Ende aufweist;
- einen zweiten Schulterträger (232), wobei der zweite Schulterträger (232) ein erstes Ende, welches über ein zweites Gurtband (244) mit dem Rückenteil (218) verbunden ist, und ein zweites Ende aufweist,
wobei das Exoskelett (200) ein flexibles Verbindungsmittel (250) aufweist, welches wenigstens eines der zweiten Enden der ersten und zweiten Schulterträger (230, 232) in Schubrichtung komprimierbar mit dem Rückenteil (218) verbindet.

2. Exoskelett (200) nach Anspruch 1,
wobei das flexible Verbindungsmittel (250) in Schubrichtung im Wesentlichen widerstandslos komprimierbar ist.

3. Exoskelett (200) nach einem der Ansprüche 1 oder 2,
wobei das flexible Verbindungsmittel (250) ein flexibles Gewebe aufweist.

4. Exoskelett (200) nach einem der Ansprüche 1 bis 3,
wobei das flexible Verbindungsmittel (250) elastisch ausgebildet ist.

5. Exoskelett (200) nach einem der Ansprüche 1 bis 4,
wobei das flexible Verbindungsmittel (250) an einem ersten Ende mit dem Rückenteil (218) und an einem gegenüberliegenden, zweiten Ende mit mindestens einem der Schulterträger (230, 232) verbunden ist, wobei das erste Ende des flexiblen Verbindungsmittels an einer dem Nutzer Abgewandten Oberfläche angebracht ist.

6. Exoskelett (200) nach einem der Ansprüche 1 bis 5,
wobei das flexible Verbindungsmittel einen netzartigen Bereich aufweist.

7. Exoskelett (200) nach einem der Ansprüche 1 bis 6,
wobei das flexible Verbindungsmittel (250) faltbar ist.

8. Exoskelett (200) nach einem der Ansprüche 1 bis 7,
wobei das flexible Verbindungsmittel (250) von einem ersten, gespannten Zustand, insbesondere bei Aufrechter Haltung des Nutzers, in einen zweiten, komprimierten Zustand, insbesondere bei gebeugter Haltung des Nutzers, überführbar ist.

9. Exoskelett (200) nach einem der Ansprüche 1 bis 8,
wobei das Exoskelett (200) verstellbare Schultergurte (206, 208) zum Anpassen des Exoskeletts (200) an die Größe des Benutzers aufweist, und wobei die verstellbaren Schultergurte (206, 208) insbesondere durch die Schulterträger (230, 232) geführt werden.

10. Exoskelett (200) nach Anspruch 9,
wobei das Exoskelett (200) wenigstens einen elastischen Expander (210, 212) aufweist, welcher zwischen wenigstens einem der verstellbaren Schultergurte (206, 208) des Exoskeletts (200) und wenigstens einem Beingurt (202, 204) angeordnet und dazu ausgebildet ist, eine Rückstellkraft zu erzeugen, wenn der Anwender, insbesondere durch eine Beugung nach vorne, einen Abstand zwischen dem Schultergurt (206, 208) und dem Beingurt (202, 204) vergrößert.

11. Exoskelett (200) nach einem der Ansprüche 1 bis 10,
wobei das Exoskelett (200) zwei Beingurte (202, 204) aufweist, welche dazu ausgebildet sind, je ein Bein des Benutzers zu umschlingen, und wobei das Exoskelett (200) einen ersten elastischen Expander (210) aufweist, welcher zwischen einem ersten verstellbaren Schultergurt (206) und dem ersten Beingurt (202) angeordnet ist, und wobei das Exoskelett (200) einen zweiten elastischen Expander (212) aufweist, welcher zwischen einem zweiten verstellbaren Schultergurt (208) und dem zweiten Beingurt (204) angeordnet ist.

12. Exoskelett (200) nach Anspruch 11,
wobei der erste Beingurt (202) über einen ersten verstellbaren Riemen (214) mit dem ersten Expander (210) verbunden ist, und wobei der zweite Beingurt (204) über einen zweiten verstellbaren Riemen (216) mit dem zweiten Expander (212) verbunden ist.

13. Exoskelett (200) nach einem der Ansprüche 1 bis 12,
wobei das Exoskelett (200) dazu ausgebildet ist, eine gebeugte Haltung und/oder ein Aufrichten des Anwenders passiv zu unterstützen.
